# EUROPEAN PATENT APPLICATION

(11) **EP 4 144 308 A1**
(43) Date of publication of application: **08.03.2023**
(21) Application number: 21195282.5
(22) Date of filing: 07.09.2021
(51) Int. Cl.: A61B 17/22, A61B 17/221, A61B 34/00

(54) **MAGNETIC DEVICE AND SYSTEM FOR URINARY STONE EXTRACTION USING MAGNET**

(71) Applicant: Srinivasan, Shyam, 80636 Munich (DE)
(72) Inventor: Srinivasan, Shyam, 80636 Munich (DE)
(74) Representative: Bertsch, Florian Oliver

(57) **Abstract**

A medical device for extraction of urinary stones, comprising:
- at least one wire extending from proximal end of the medical device towards a distal end of the medical device, and
- a magnet arranged at the distal end and coupled to the at least one wire and configured to provide a stray magnetic field in a surrounding to attract magnetic particles attached to the urinary stones.

## Description

### TECHNICAL FIELD

Various examples of the disclosure generally relate to techniques for extracting urinary stones from a urinary tract of a patient. Various examples of the disclosure specifically relate to a medical device including a magnet for attracting magnetized urinary stones.

### BACKGROUND

Various devices are known for removing objects from the human body. For example, kidney stones, gallstones, and the like can be removed from the respective tract.

To remove urinary stones, various techniques are known. A surgical technique uses an endoscope that is inserted into the urinary tract. The endoscope has a lumen and a retrieval module can be delivered to a distal end of the endoscope through the lumen. For instance, a wire basket can be used to retrieve, retrain and subsequently extract the urinary stones.

The following prior art is known: US20090136594A1; WO2016148747A1; CN105025823B; US20080045881A1; US4554088A; WO2017054749A1; CN1098896A; US7066924B1; CN2031656U; US6902528B1; CN201323106Y; CN202051675U; EP2796101B1; CN106552296A; US9925311B2; Tracy, Chad R., et al. "Improving The Efficiency of Stone Fragment Removal: Use Of Iron-Oxide Microparticles To Allow Stone Extraction With Novel Magnetic Tools." The Journal of Urology 181.4S (2009): 664-664; Elsayed, Hany H., et al. "A magnet built on bronchoscopic suction for extraction of tracheobronchial headscarf pins: a novel technique and review of a tertiary centre experience." Interactive cardiovascular and thoracic surgery 22.5 (2016): 531-536; Tan, Y. K. e. a. Novel iron oxide microparticles used to render stone fragments paramagnetic: assessment of toxicity in a murine model. s.l. : The Journal of urology 188.5: 1972-1977, 2012; Markides, H., M. Rotherham, and A. J. El Haj. Biocompatibility and toxicity of magnetic nanoparticles in regenerative medicine. s.l. : Journal of Nanomaterials 2012, 2012.

### SUMMARY

A need exists for advanced techniques of retrieving urinary stones or other calculi. This need is met by the features of the independent claims. The features of the dependent claims define embodiments.

According to one aspect a medical device for extraction of urinary stones is provided, the medical device comprising at least one wire extending from a proximal end of the medical device towards a distal end of the medical device. The medical device further comprises a magnet arranged at the distal end and coupled to the at least one wire and configured to provide a stray magnetic field in a surrounding to attract magnetic particles attached to the urinary stones.

Furthermore a method is provided comprising the steps of attracting and retaining (3030) urinary stones from a target region using a magnet attached to a wire included in a lumen of an endoscope.

It is to be understood that the features mentioned above and those yet to be explained below may be used not only in the respective combinations indicated, but also in other combinations or in isolation without departing from the scope of the invention.

### BRIEF DESCRIPTION OF THE DRAWINGS

FIG. 1 schematically illustrates a medical device according to various examples, the medical device including a magnet attached to the wire or staff/rod.
FIG. 2 schematically illustrates a ferromagnetic core of the magnet integrated with a wire basket according to various examples.
FIG. 3 schematically illustrates a ferromagnetic core of the magnet arranged adjacent to and offset from a wire basket according to various examples.
FIG. 4 schematically illustrates a ferromagnetic core of the magnet being arranged inside a wire basket according to various examples.

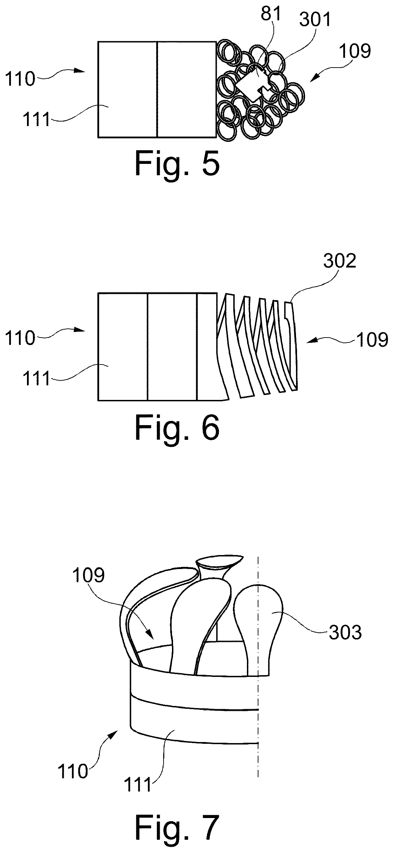

FIG. 5 schematically illustrates a retention feature for retaining urinary stones at a magnetic apex of the magnet according to various examples.
FIG. 6 schematically illustrates a retention feature for retaining urinary stones at a magnetic apex of the magnet according to various examples.
FIG. 7 schematically illustrates a retention feature for retaining urinary stones at a magnetic apex of the magnet according to various examples.
FIG. 8 schematically illustrates a retention feature for retaining urinary stones at a magnetic apex of the magnet according to various examples.
FIG. 9 schematically illustrates a retention feature for retaining urinary stones at a magnetic apex of the magnet according to various examples.
FIG. 10 schematically illustrates a retention feature for retaining urinary stones at a magnetic apex of the magnet according to various examples.
FIG. 11 schematically illustrates a ferromagnetic pseudo-wire according to various examples.
FIG. 12 schematically illustrates an implementation of the magnet including a ferromagnetic core and an electric coil according to various examples.
FIG. 13 schematically illustrates a hysteretic behavior of a ferromagnetic core according to various examples.
FIG. 14 schematically illustrates a system including the medical device and an endoscope according to various examples.
FIG. 15 schematically illustrates aspects with respect to an integration of the magnet in the endoscope according to various examples.
FIG. 16 is a flowchart of a method according to various examples.

### DETAILED DESCRIPTION

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

Various examples of the disclosure generally relate to surgical techniques for extracting objects, e.g., urinary stones, from the urinal tract of a patient. An endoscope can be used to deliver a medical device configured to collect, retain, and extract the urinary stones.

A medical device for extraction of urinary stones includes a wire extending from a proximal end to a distal end of the medical device. The medical device also includes a magnet. The magnet is arranged at the distal end and is configured to provide a stray magnetic field in its surrounding to attract magnetic particles that are attached to the urinary stones.

Thus, by means of the stray magnetic field, urinary stones such as kidney stones can be collected and, at least to some degree, retained.

For instance, techniques for attaching magnetic particles to kidney stones, to thereby magnetize the kidney stones, are known from US9925311 B2 or European patent application 20160490.7. Such and other techniques may be used in an extraction process disclosed herein.

The magnet can thus attract and retain - e.g., magnetically attached to its surface - kidney stones that have been magnetized.

The magnet may be displaced towards the distal or proximal end by means of the wire. Thereby, it is possible to advance the magnet towards urinary stones, e.g., even where an endoscope remains static.

A magnetic apex of the magnet may be provided that is configured to engage the urinary stones. The magnetic apex may present an engagement surface. The engagement surface can be progressed through a target region of the urinal tract of the patient. The engagement surface may be rounded to avoid a trauma (abated risk of damage to kidney tissue -sharp contours in baskets and aspirators cause abrasions).

Once the urinary stones are attracted and arranged adjacent to the engagement surface, the magnet may be retracted from the target region, e.g., conveyed through a lumen of the endoscope to the proximal end of the endoscope. Thereby, the urinary stones can be extracted.

As a general rule, the magnet can be coated. For instance, a biocompatible polymer can be used to protect the urinary tissue from sharp metal contours.

As a general rule, the magnet can have a diameter that is dimensioned to move through an endoscope lumen. For instance, a diameter of the magnet can be as large as 2.5 mm. This enables to introduce and retract the magnet during the extraction procedure. In particular, the extraction using the magnet can be combined with other techniques for urinary stone extraction that are enabled by the endoscope.

As a general rule, a diameter of the magnet may be in the range of 0.5 to 4 mm or 7 mm in case of PCNL, if introduced retroactively.

The magnet can include a ferromagnetic core. The ferromagnetic core can be cylindrical in shape. The ferromagnetic core may be formed by a ferromagnetic material. The ferromagnetic core may or may not be surrounding by an electric coil of the magnet. The ferromagnetic core can be implemented by soft or hard ferromagnetic materials. Examples of soft ferromagnetic materials include: iron; low carbon steel; magnetic stainless steel; silicon iron; cobalt iron; nickel alloy; permalloy; Mu Metal. Examples of hard ferromagnetic materials include: Alnico; sintered NdFeB; ceramic; sintered SmCo; bonded NdFeB. The ferromagnetic core can include single-molecule magnets (SMMs) and/or single-chain magnets (SCMs) and/or nano-structured magnets. The ferromagnetic core could include an alloy consisting of Nickel, Iron and Molybdenum. A permeability of 200.000 to 250.000 may be used. The ferromagnetic core can be implemented by a soft ferromagnet alloy, e.g., permalloy. A ferromagnetic shape memory alloy could be used. For example, the ferromagnetic core can be formed by a pseudo-wire of magnetic dipoles; here, the magnetic dipoles can be arranged sequentially.

Alternatively or additionally to the ferromagnetic core, the magnet can include an electric coil. An excitation current - delivered through a respective wire - can be used to generate a magnetic field. This magnetic field can, in some examples, be amplified by polarizing or de-polarizing the ferromagnetic core.

As a general rule, excitation currents in the range of 20 mA to 10 A would be possible.

As will be appreciated from these explanations, there are various options available for implementing the magnet and some are explained in connection with TAB. 1.

**TAB. 1: Various options for implementing and operating the magnet. Where an intracorporeal excitation current is used, an electromagnetic shielding can be used, e.g., to shield a camera sensor from interference.**

| | Option | Description |
|---|---|---|
| I | Pre-magnetized ferromagnet | The ferromagnet can be extracorporeally pre-magnetized. For instance, a system can include the magnetic device and an external magnetic field generator, e.g., a magnetic coil. Then, the ferromagnet can be magnetized to saturation outside of the patient's body. Once the polarizing magnetic field is removed, the ferromagnet relaxes to remanence. This remanence magnetic field can be used to attract and retain magnetic particles when the magnet is arranged intracorporeally. |
| II | Actively magnetized ferromagnet | The ferromagnet could also be magnetized in vivo. An electric coil can be used to polarize or de-polarize the ferromagnetic core. For instance, such electric coil could have a biocompatible polymer coating. |
| | | By means of the electric coil, the strength of the stray magnetic field can be switched intracorporeally. The excitation current can be provided by a control unit. The control unit can include an digital-to-analog converter of a power supply unit to provide the excitation current. The excitation current can be dimensioned to polarize or de-polarize the magnetization of the ferromagnetic core in a hysteretic regime of the ferromagnetic core, e.g., between coercivity and remanence. |
| | | An alternating-current (AC) excitation can be used, e.g., to weaken the net magnetic flux density of the ferromagnetic core (de-polarize). By switching the current direction, the ferromagnetic core can be polarized or de-polarized selectively. |
| III | Electromagnet | An electric coil can be used without ferromagnetic core. The excitation current can be delivered to the electric coil when the magnet is placed at the extraction region. |
| | | For instance, such electric coil could have a biocompatible polymer coating. As a general rule, a coating can be used that protects the electric coil against fluids, e.g., introduced through another lumen of the endoscope. |

In some scenarios, the medical device could further include a wire basket that is arranged at the distal end. The wire basket can be configured to retrieve and retain the urinary stones when the wire basket is actuated using the at least one wire.

As a general rule, a magnetic apex of the magnet may include one or more retention features such as a wire basket. These retention features can trap urinary stones such as kidney stones.

Such techniques facilitate attracting magnetized stone dust while still enabling to catch larger urinary stones using the wire basket. Retrieving and retaining urinary stones is a well-established surgical procedure. Magnetic extraction is thereby added to the well-established surgical procedure, which facilitates attracting and retaining smaller kidney stones that would normally be left behind; such left-behind kidney stones bear the risk of growing into new, larger stones. The same is true for other calculi.

The present application will be explained in in the context of kidney stones in the following. However it should be understood that the technique discloses can be used in connection with other calculi or concrements with biomineral, mineral or crystalline surfaces including bilirubin and uric acid. Example wire baskets are grasping forceps, no-tip nitinol baskets, and stainless steel baskets.

Various techniques are possible for implementing the wire basket. The wire basket can include a plurality of capture wires. The plurality of capture wires can form the wire basket. The wire basket can include a wire collecting element. The wire collecting element could be arranged at the distal end of the wire basket. The wire collecting element can have a substantially rigid body and can receive the capture wires. The wire collecting element can secure the capture wires at the distal end of the wire basket. Thereby, a substantially tipless basket can be provided.

For instance, the wire basket and the magnet can be used to retrieve kidney stones of different sizes. Specifically, it would be possible that comparably small kidney stones can be retrieved by the magnet and subsequently extracted from the urinary tract, if compared to sizes of kidney stones that can be retrieved, retained, and extracted using the wire basket. For instance, typical sizes of kidney stones that can be retrieved and retained by the wire basket can be in the range of 10 µm to 5 mm: smaller kidney stones may escape through the wire grid formed by the capture wires. Differently, kidney stones having sizes as small as a few nanometers, e.g., 5 nanometers, may be attracted and retained by the magnet, and subsequently extracted. This has the advantage that by also extracting such small kidney stones, it is avoided that such small kidney stones serve as seed points for subsequent kidney stone growth.

As a general rule, various options are available for combining, in the retrieval process, operation of the wire basket and operation of the magnet. Some options are summarized below in TAB. 2.

**TAB. 2: Various options for operating a wire basket in combination with a magnet for retrieving kidney stones.**

| Option | Brief description | Example details |
|---|---|---|
| I | Sequential operation | For example, it would be possible to first retrieve at least parts of the kidney stones using the wire basket. For instance, larger kidney stones can be retrieved. |
| | | Then, upon retrieving at least the parts of the kidney stones using the wire basket, residual kidney stones in the urinary tract can be retrieved using the magnet. For instance, the residual kidney stones can be magnetized after operating the wire basket. |
| | | The wire basket may be retracted from a target region in the urinary tract before the magnet is advanced towards the target region. The same lumen of an endoscope may be re-used for the wire basket and the magnet. |
| | | Such approach facilitates a prior-art extraction procedure using the wire basket in combination with a magnetic extraction. |
| II | Parallel operation | For example, it would be possible to firstly magnetize the kidney stones and then advance, to the target region, both, the wire basket, as well as the magnet. Then, it would be possible to magnetically attract the magnetized kidney stones towards the wire basket such that the magnetized kidney stones can be retrieved and retained by the wire basket. Alternatively or additionally, the wire basket can be conventionally advanced towards the kidney stones to collect the kidney stones. |
| | | Thus, both the wire basket as well as the magnet may be arranged at the target region at the same point in time. |
| III | Combined operation | It would be possible that the wire basket is itself magnetic. Then, the stray magnetic field of the magnet can be used to magnetize the magnetic parts of the wire basket, e.g., magnetic capture wires, e.g., axially or diametrically. This facilitates a magnetic trap to retain kidney stones inside the wire basket. Kidney stones can be collected and retained reliably. |

For scenarios II and III, various relative arrangements of the magnet with respect to the wire basket are possible. For instance, the magnet could be arranged adjacent to the wire basket, e.g., offset towards the proximal end of the medical device. Alternatively, it would be possible that the magnet is arranged inside the wire basket.

As a general rule, the wire basket and the magnet may be attached to the same wire or different wires. According to some examples, the relative position of the wire basket with respect to the magnet may be adjusted by actuating a wire of, e.g., the magnet or the wire basket. Where different wires are used, the basket wire can be actuated without displacing the magnet.

The operation of a system including an endoscope and the medical device can be as follows. First, the endoscope is introduced into the urinary tract towards an extraction region where kidney stones are located. "Endoscope" refers to all flexible, semi-flexible and rigid endoscopes that includes ureteroscopes and ureterorenoscopes. Then, the kidney stones can be locally magnetized. For instance, a fluid for magnetization of the kidney stones can be introduced. A lumen of the endoscope can be used, e.g., a working channel of the endoscope or an access sheath such as an ureteral access sheat. Next, the magnet can be introduced through a lumen of the endoscope, from the proximal end to the distal end, towards the extraction region. Optionally, a wire basket may be introduced to the extraction region, e.g., using the same or another lumen as the lumen used to introduce the magnet. Sometimes, the wire basket may be coupled to the magnet and/or arranged adjacent to the magnet; then, the magnet and the wire basket can be introduced together.

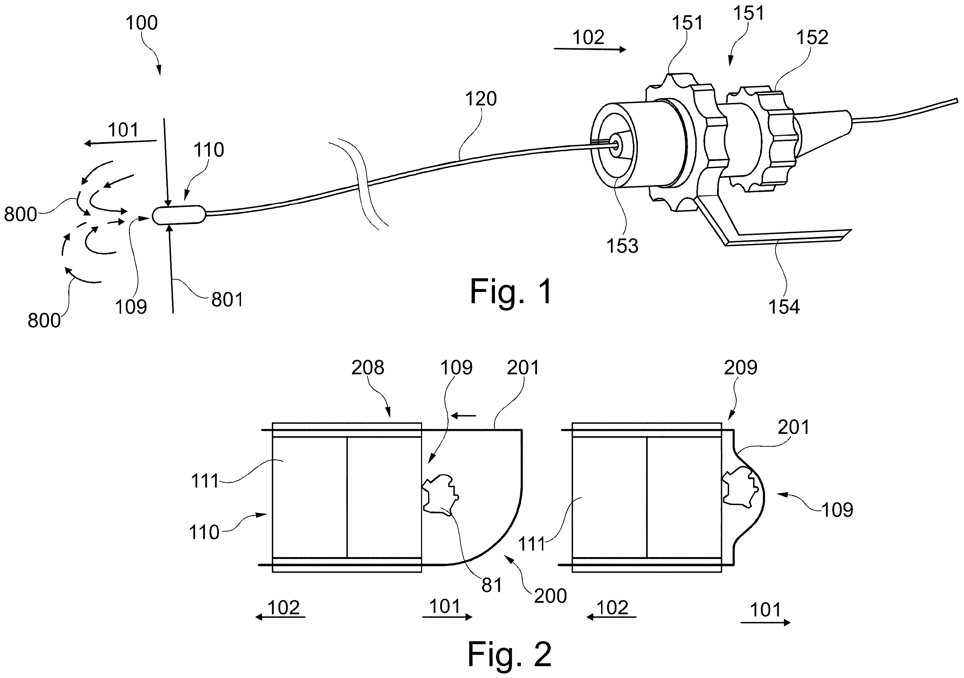

FIG. 1 schematically illustrates a medical device 100 according to various examples. The medical device 100 can be used in conjunction with an endoscope (not shown).

The medical device 100 includes a wire 120 that extends from a proximal end 102 of the medical device 100 towards a distal end 101 of the medical device 100. Also provided as a magnet 110 that is arranged at the distal end 101. The magnet 110 is coupled to the wire 120. The magnet 110 is configured to provide a stray magnetic field 800 in its surrounding. Thereby, magnetic particles attached to kidney stones, specifically magnetized urinary/kidney stones, can be attracted. A magnetic field gradient of the stray magnetic field 800 can be used to dislocate the magnetized kidney stones towards the magnet 110.

The diameter 801 of the magnet 110 can be small enough - e.g., smaller than 2.5 mm - so that the magnet 110 can be inserted into a lumen of the endoscope at its proximal end and moved through the lumen towards a distal end of the endoscope. By moving the wire 120 with respect to the endoscope, the magnet 110 can be advanced towards or retracted from a target region. I.e., the wire 120 can displace the magnet 110.

For this purpose, a connector 151 is provided. The connector 151 is configured to fix the wire 122 the endoscope at the proximal end 102. The connector 151 includes an adjustment screw 151 as well as a Luer lock 153; also, a mounting lock 152 is provided. The adjustment screw 151 can be actuated via a respective handle 154. This enables one-handed operation: once the magnet 110 has been positioned at a desired axial location, this axial location can be fixed by one-handed operation of the Luer lock 153.

According to various examples, the medical device 100 may further include a wire basket. An example implementation of a combination of the magnet 110 and the wire basket is illustrated in connection with FIG. 2.

FIG. 2 schematically illustrates aspects with respect to a combination of a magnet 110 and a wire basket 200. FIG. 2 is a cross-sectional view.

FIG. 2 schematically illustrates a scenario in which the magnet 110 and the wire basket 200 are integrated. In detail, the magnet 110 includes a ferromagnetic core 111. Capture wires 201 of the wire basket 200 are axially aligned with the ferromagnetic core 111 and run along a circumference of the ferromagnetic core 111.

FIG. 2 schematically illustrates the wire basket 200 in an open position 208 in a closed position 209. A magnetized kidney stone 81 is attracted towards a magnetic apex 109 of the magnet 110. The magnetic apex 109 can engage the urinary/kidney stones 81. To retain the kidney stone 81, it is then possible to tighten the capture wires 201, so as to close the wire basket 200, which defines the closed position 209.

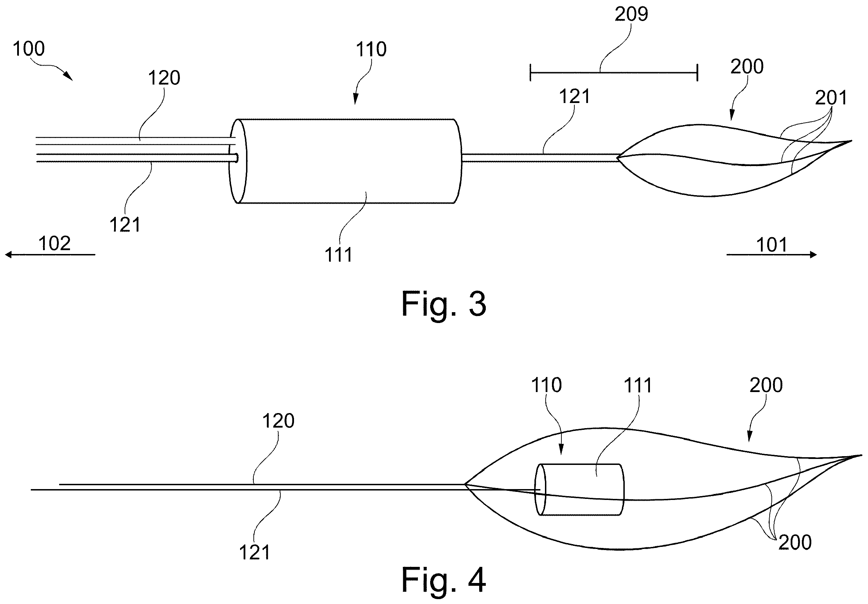

The arrangement of the wire basket 200 with respect to the magnet 110 illustrated in FIG. 2 is only one example. Other examples are possible. For instance, as illustrated in FIG. 3, it would be possible that the magnet 110 is arranged adjacent but offset from the wire basket 200. A respective offset 209 is illustrated.

Also illustrated in FIG. 3 is a scenario in which the magnet is attached to the wire 120; while a further wire 121 is attached to the wire basket 200 and can be used to open and close, i.e., actuate between the open and closed positions 208, 209, the wire basket 200. Thereby, the magnet 110 may be advanced towards or retracted from the distal end 101 independent of the wire basket 200; the magnet 110 can be displaced towards or away from the wire basket 200.

Yet another scenario of the relative arrangement of the magnet 110 with respect to the wire basket 200 is illustrated in FIG. 4. In the scenario of FIG. 4, the magnet 110 is arranged inside the wire basket 200.

Yet another scenario of the relative arrangement of the magnet 110 with respect to the wire basket 200 is illustrated in FIG. 4. In the scenario of FIG. 4, the magnet 110 is arranged inside the wire basket 200.

The wire basket 200 thus retains the kidney stones 81 at the magnetic apex 109 of the magnet 100. Thereby, the kidney stones 81 can be extracted reliably.

As a general rule, various kinds and types of retention features would be possible, also different than the wire basket 200. Some examples are disclosed below in TAB. 3.

**TAB. 3: Various examples of retention mechanisms that can be used to fix and retain kidney stones that have been attracted by the magnet 110 at the magnetic apex 109. By means of the retention mechanism, it is possible to reliably extract kidney stones. It is avoided that the kidney stones are brushed of the magnetic apex 109 when retracting the magnet 110 through a lumen of the endoscope towards the proximal end 102.**

| Option | Example description | Example details |
|---|---|---|
| I | Wool grain | As illustrated in FIG. 5, it would be possible to use wool grain such as steel wool to entangle the kidney stones 81. The wool 301 can be magnetized using the magnet 110. |
| II | Compression spring | As illustrated in FIG. 6, a compression spring 302 can be used to engage and retain kidney stones 81. Again, it would be possible to magnetize the compression spring 302 using the magnet 110. |
| III | Flaps | As illustrated in FIG. 7, flaps can be attached to the magnetic apex 109 of the magnet 110 and can be used to retain kidney stones 81. The magnetic flaps 303 could be magnetized by the magnet 110. By changing the stray magnetic field, the flaps 303 can be bent to the closed position or to the open position. The magnetic flaps 303 may be actuated between an open position 208 and a closed position 209, cf. FIG. 8. |
| IV | Indent | As illustrated in FIG. 9, the magnetic apex 109 may have an indent 305 or cavity so that magnetized kidney stones 81 can accumulate inside the indent 305. |
| V | Hooks | Illustrated in FIG. 10 is an implementation of the retention mechanism relying on hooks 306 that can engage the magnetized kidney stones 81. |
| VI | Wire basket | The wire basket discussed above in connection with FIG. 2, FIG. 3 and FIG. 4 is a further example of retention mechanism. |
| | | Capture wires of the wire basket may be made from a ferromagnetic material. Thereby, the capture wires can be magnetized. |

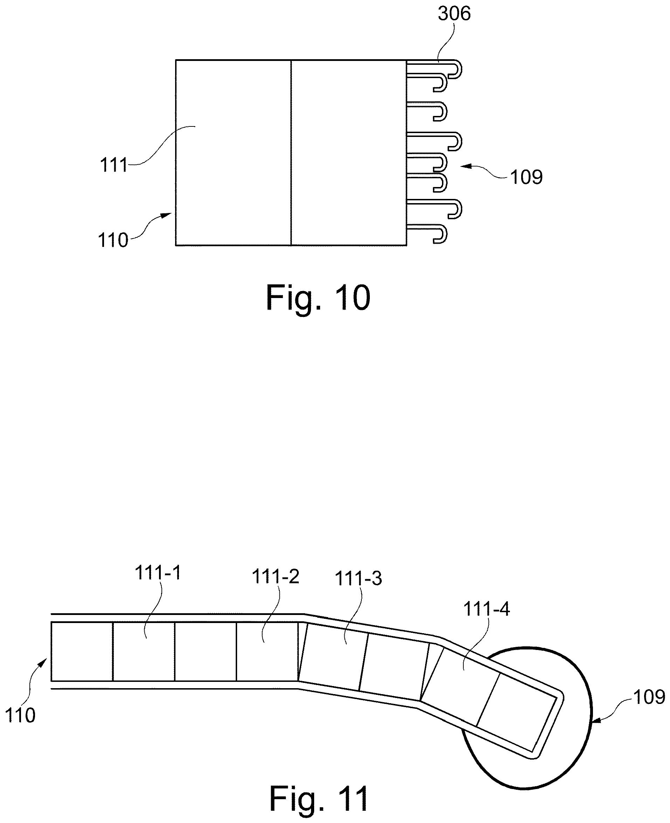

While above implementations of the magnet 110 using a ferromagnetic core 111 implemented as magnetic dipole are shown, as a general rule, other implementations would be possible. One example implementation as illustrated in FIG. 11. Here, multiple magnetic dipoles 111-1 ― 111-4 are arranged in sequence, e.g., in an elastic tube. This forms a magnetic pseudo-wire. Such ferromagnetic pseudo-wire can be flexibly combined with a retention mechanism as discussed above.

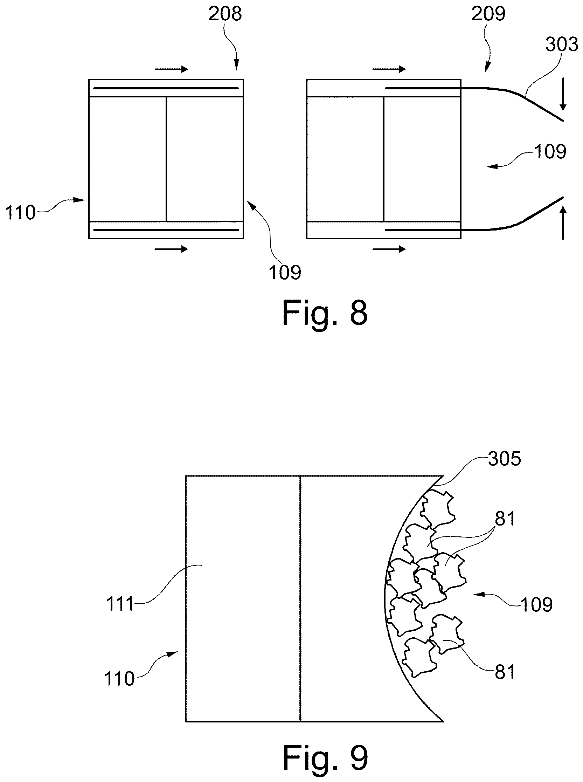

In various examples, it can be helpful to adjust the strength of the stray magnetic field 800. For instance, adjusting the strength of the stray magnetic field can be helpful to switch a retention feature between the closed position and the open position (cf. FIG. 8). For instance, adjusting the strength of the stray magnetic field can be helpful to selectively attract kidney stones, e.g., where a parallel or combined operation of the magnet and a wire basket is used (cf. TAB. 2). Various options are available for adjusting the strength of the stray magnetic field 800. For instance, it would be possible to change the offset between a ferromagnetic core 111 of the magnet 110 and a retention feature. For instance, it would be possible to display the ferromagnetic core 111 towards or away from a target region. Such displacement can be achieved using a wire 120. Another option would be to change an excitation current of an electric coil (cf. TAB. 1, example III); or to polarize/de-polarize a ferromagnetic core (cf. TAB. 1, example II).

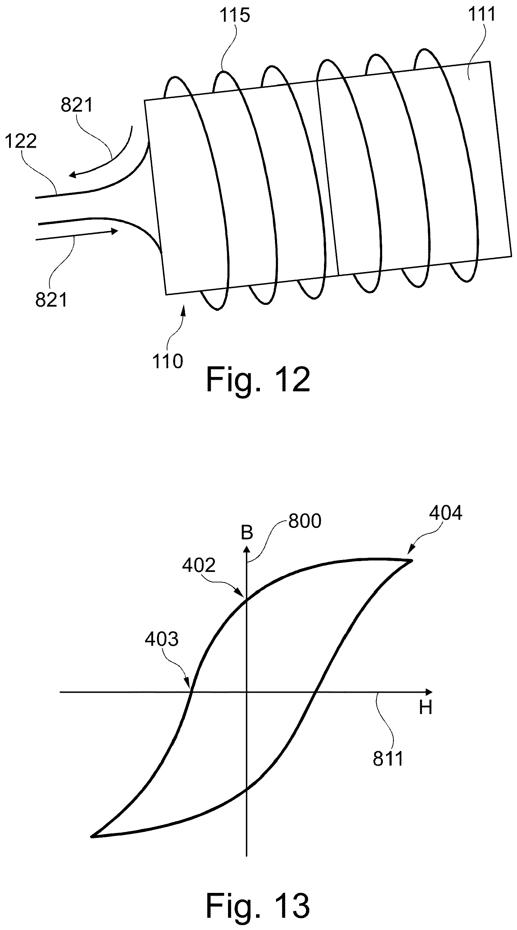

FIG. 12 illustrates aspects with respect to the magnet 110. In the illustrated example, the magnet 110 includes an electric coil 150 that is wound about the ferromagnetic core 111. A wire 122 can be electrically coupled to the electric coil 115 to deliver in excitation current for polarizing or depolarizing the ferromagnetic core 111 of the magnet 110. for instance, it would be possible that the excitation current is dimensioned so as to switch between remanence 402 and coercive field 403 (cf. FIG. 13, where a hysteretic behavior of the stray magnetic field 800 as a function of the polarizing/depolarizing external magnetic field 811 is illustrated). Here, large changes of the stray magnetic field 800 can be enabled with comparably small external magnetic fields. Typical magnetic field strengths at remanence 402 may be in the range of 60 mT to 250 mT.

For instance, it has been found that there can be a tendency of capturing more kidney stones for larger magnetic fields. For instance, simulations have shown that it is possible to remove 97 % of the calcium oxalate fragments with a magnetic field of 184 mT.

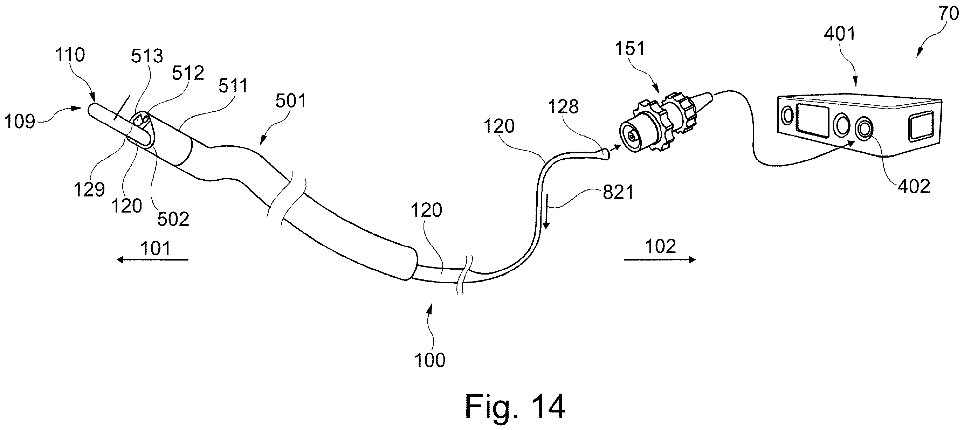

FIG. 14 illustrates aspects with respect to a system 70. The system 70 includes the medical device 100, as well as a control unit 41 that is configured to apply the excitation current 821. The control unit 41 can be configured to apply the excitation current 821 in the hysteretic regime of the ferromagnetic core 111 of the magnet 110 between coercivity 403 and remanence 402.

The control unit 41 can be implemented by electronic circuitry that enables the limitation and adjustment of voltage, current, pulse width, frequency and bursts.

The excitation current 821 can be at least in part supported/conducted by the wire 120 which the magnet 110 is attached (the wire 120 may be electrically isolated). As a general rule, the same wire may be used to displace the magnet and to provide the excitation current. It would also be possible to use different wires.

As illustrated in FIG. 14, the system 70 also includes an endoscope 501. The endoscope 501 includes a lumen 502 extending between the proximal end 102 and the distal end 101 and the magnet 110 is dimensioned to move through the lumen 502 from the proximal end 102 to the distal end 101. the endoscope 51 could also include a camera sensor 513 for image-based guidance.

Alternatively or additionally, it would also be possible that a magnetic sensor is provided, adjacent to the magnet 110, e.g., at the distal end 101 of the endoscope 501. Then, it would be possible that the control unit 401 is configured to apply the excitation current 821 using a feedback control loop. The measured output of the feedback control loop can be determined based on the sensor signal of the magnetic sensor. This is particularly helpful for hysteretic switching (cf. FIG. 13) where non-linear behavior is observed.

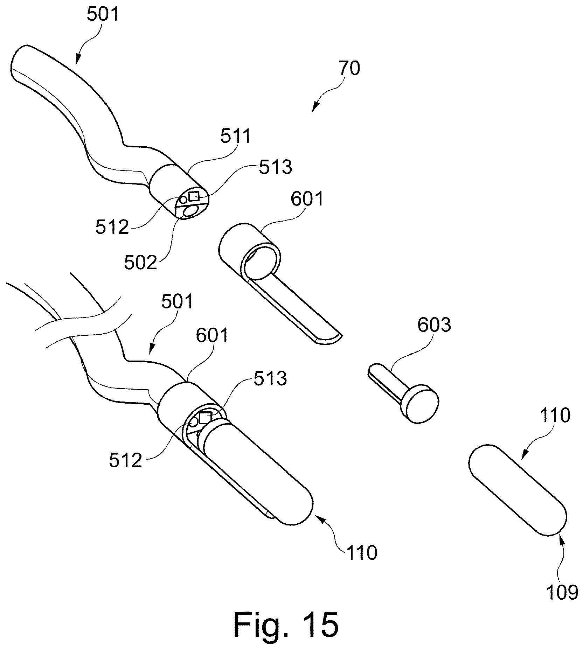

FIG. 15 is an exploded schematic view of the system 70. Illustrated is the attachment of the magnet 110 via an attachment element 601 and a mount 603.

The attachment element 601 can be used to hold the magnet 110 in front of the distal end of the endoscope.

The mount 603 can be used to provide a current to an electric coil (not shown in FIG. 15) of the magnet 110.

A heat sink (not shown) may be provided to absorb heat induced by polarizing /depolarizing a ferromagnetic core.

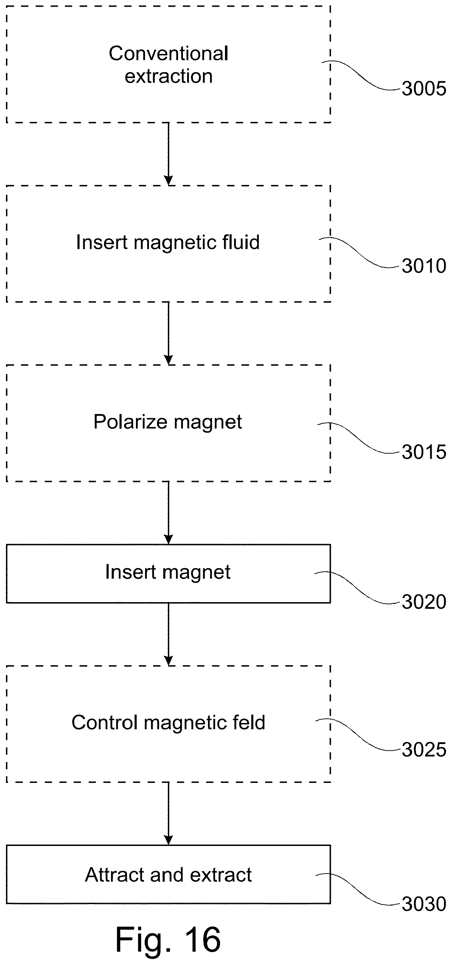

FIG. 16 is a flowchart of a method according to various examples. FIG. 16 illustrates an extraction procedure for extracting kidney stones from a target region in the urinary tract of a patient. Optional boxes are illustrated with dashed lines.

At optional box 3005, a conventional extraction can be implemented. Here, it would be possible that a wire including a basket at its distal end is inserted into a lumen of an endoscope such as the endoscope 501 discussed above in connection with FIG. 14 and FIG. 15. The wire basket can be advanced towards the distal end of the endoscope. Then, kidney stones can be captured and retained by the wire basket.

Subsequently, the wire basket - in its closed position - can be retracted through the lumen of the endoscope, thereby extracting the captured kidney stones.

It would then be possible, at box 3010, to insert a magnetic fluid into the target region. This could be achieved using the lumen of the endoscope once the wire including the wire basket has been removed. The magnetic fluid can include magnetic particles that can adhere to the surface of the kidney stones, thereby magnetizing the kidney stones.

At box 3015, a ferromagnetic core of a magnet of a medical device according to various examples can be extracorporeally polarized. For this, the magnet can be placed in a polarizing magnetic field. The polarizing magnetic field could be generated by a coil or a permanent magnet.

It is not required in all scenarios to pre-polarized the ferromagnetic core at box 3015. For instance, as explained above in connection with TAB. 1, it would be possible that the magnet does not include a ferromagnetic core, but is implemented by an electromagnet (cf. TAB. 1: example III). It would also be possible that the magnet includes a ferromagnetic core, however, the ferromagnetic core may be actively magnetized intracorporeally (cf. TAB. 1: example II). As such, box 3015― implementing example I of TAB. 1 - is optional.

Next, at box 3020, the magnet can be inserted using a lumen of the endoscope. For instance, the lumen of the endoscope that is now vacated and has been previously used for the conventional extraction at box 3005 and/or the insertion of the magnetic fluid at box 3010 may be used. The location of the magnet with respect to the target region can be controlled by displacement of the respective wire (cf. FIG. 1; connector 151). The magnet has a diameter which is small enough so that the magnet can be advanced through the lumen of the endoscope from the proximal end all the way to the distal end of the endoscope.

It is optionally possible, at box 3025, to control the strength of the stray magnetic field at the target region. Various options are available for controlling the strength of the stray magnetic field at the target region. In one option, the strength of the stray magnetic field can be controlled by controlling the location of the magnet with respect to the target region. For instance, the magnet can be advanced or retracted from the target region, thereby increasing or decreasing the strength of the stray magnetic field. A wire to which the magnet is attached can be moved with respect to the endoscope using a respective connector (cf. FIG. 1 and FIG. 14: connector 151). In another option, the strength of the stray magnetic field can be controlled by polarizing or depolarizing the ferromagnetic core, as explained previously in connection with FIG. 12 and FIG. 13. For instance, the excitation current 821 can be dimensioned to switch between the remanence and coercive field of the ferromagnetic core 111. It would be possible that a closed-loop control is implemented using a sensor signal obtained from a magnetic field sensor arranged at the distal end of the endoscope 501.

Then, at box 3030, it is possible to attract, retain, and extract urinary/kidney stones using the stray magnetic field. Specifically, it would be possible that /urinary kidney stones are pulled towards the magnet using the magnetic force induced by the stray magnetic field. Further, the kidney stones can be retained at the magnetic apex of the magnet using a retention feature, e.g., a basket or another retention feature as discussed above in connection with TAB. 3. Thus, as will be appreciated, according to various examples, it would be possible to combine operation of a structural retention features such as a wire basket with magnetic extraction using the magnet (cf. TAB. 2: example II and example III).

Generally, using the extraction at box 3005 and the extraction at box 3010, kidney stones of different size regimes can be extracted. For instance, at box 3005 kidney stones having a size in the range of 10 µm to 5 millimeter may be extracted; while, at box 3030, kidney stones having sizes in the range of 5 nanometer to 2 millimeter may be extracted.

Various modifications of the method of FIG. 16 would be conceivable. For instance, it would be possible that different sequences of the various boxes are implemented. For instance, the magnetic fluid at box 3010 may be inserted before an attempt of conventional extraction is made at box 3005. It would also be possible that conventional extraction at box 3005 is implemented after box 3030. According to examples, it would even be possible that conventional extraction using a basket at box 3005 is implemented in parallel to box 3030; for this purpose, an endoscope may be used that includes two lumen and two wires, one attached to a basket, and another one attached to magnet (and, optionally, a retention feature such as a further wire basket) may be advanced through the two lumen.

From the above said some general conclusions can be drawn for the medical device:
Preferably, the magnet (110) comprises a magnetic apex (109) configured to engage the urinary stones (81), and more preferably the magnetic apex comprises one or more retention features (200, 301-307) configured to engage and retain the urinary stones (81).
Preferably the magnet comprises a ferromagnetic core (111, 111-1 - 111-4) and more preferably the ferromagnetic core is formed by a pseudo-wire of magnetic dipoles (111-1 ― 111-4).

As far as the method is concerned, the steps are provided attracting and retaining (3030) urinary stones from a target region using a magnet attached to a wire included in a lumen of an endoscope. Preferably, the method further comprises the steps of capturing and extracting (3005) further urinary stones from the target region of a urinary tract of a patient using a wire basket.

The further urinary stones have sizes in the range of 10 µm to 5 mm, and the urinary stones have sizes in the range of 5 nm to 2 mm.

Although the invention has been shown and described with respect to certain preferred embodiments, equivalents and modifications will occur to others skilled in the art upon the reading and understanding of the specification. The present invention includes all such equivalents and modifications and is limited only by the scope of the appended claims.

For illustration, various scenarios have been disclosed that provide extraction techniques for extracting kidney stones. Respective techniques may also be applied for extracting other intracorporeal objects.

## Claims

1. A medical device (100) for extraction of urinary stones (81), comprising:
- at least one wire (120, 121, 122) extending from proximal end (102) of the medical device (100) towards a distal end (101) of the medical device (100), and
- a magnet (110) arranged at the distal end (101) and coupled to the at least one wire (120, 121, 122) and configured to provide a stray magnetic field (800) in a surrounding to attract magnetic particles attached to the urinary stones (81).

2. The medical device (100) of claim 1, further comprising:
- a wire basket (200) arranged at the distal end (101) and configured to retrieve (201) and retain (202) the urinary stones (81) when being actuated using the at least one wire (120, 121, 122).

3. The medical device of claim 2,
wherein the wire basket (200) comprises magnetizable capture wires (201) arranged in the surrounding of the magnet (110).

4. The medical device of claim 2 or 3,
wherein the magnet (110) is arranged adjacent to the wire basket (200) offset (209) towards the proximal end (102) or the magnet (110) is arranged inside the wire basket (200).

5. The medical device of any one of claims 2 to 4,
wherein a first wire (120) of the at least one wire (120, 121, 122) is configured to actuate the wire basket (200),
wherein a second wire (121) of the at least one wire (120, 121, 122) is configured to displace the magnet (110) towards or away from the wire basket (200).

6. The medical device of any one of the preceding claims,
wherein the magnet (110) comprises a magnetic apex (109) configured to engage the urinary stones (81), and preferably the magnetic apex comprises one or more retention features (200, 301-307) configured to engage and retain the urinary stones (81).

7. The medical device of any one of the preceding claims,
wherein the at least one wire (120, 121, 122) is configured to displace the magnet (110) towards the distal end (101) or towards the proximal end (102).

8. The medical device of any one of the preceding claims,
wherein the magnet (110) comprises an electric coil (115),
wherein the at least one wire (122) is electrically coupled to the electric coil (115) to deliver an excitation current for polarizing or de-polarizing a ferromagnetic core (111, 111-1 ― 111-4) of the magnet (110).

9. A system (70), comprising:
- the medical device of claim 8, and
- a control unit (401) configured to apply the excitation current (821) to the at least one wire.

10. The system of claim 9,
wherein the control unit (401) is configured to apply the excitation current (821) in a hysteretic regime of the ferromagnetic core (111, 111-1 - 111-4) between coercivity (403) and remanence (402).

11. The system of claim 9 or 10, further comprising:
- a magnetic sensor arranged in the surrounding of the magnet,
wherein the control unit is configured to apply the excitation current using a feedback control loop, wherein a measured output of the feedback control loop is determined based on a sensor signal of the magnetic sensor.

12. A system, comprising:
- the medical device (100) of any one of claims 1 to 8, and
- an endoscope (501) comprising a lumen (502) extending between the proximal end (102) and the distal end (101),
wherein the magnet (110) is dimensioned to move through the lumen.

13. The system of claim 12,
wherein the endoscope comprises a further catheter lumen extending in parallel to the catheter lumen,
wherein the system further comprises:
- a further wire extending through the further catheter lumen,
- a further wire basket arranged at the distal end at the distal end (101) and configured to retrieve (201) and retain (202) the urinary stones (81) when being actuated using the further wire.

14. The system of claim 12 or 13, further comprising:
- a connector (151) configured to fix the at least one wire to the endoscope (501) at the proximal end (102), wherein the connector comprises a Luer lock.

15. A method, comprising:
- attracting and retaining (3030) urinary stones from a target region using a magnet attached to a wire included in a lumen of an endoscope.
